# EUROPÄISCHE PATENTANMELDUNG

(11) **EP 2 036 875 A1**
(43) Veröffentlichungstag der Anmeldung: **18.03.2009**
(21) Anmeldenummer: 07116155.8
(22) Anmeldetag: 11.09.2007
(51) Int. Cl.: C07C 29/80, C07C 35/12

(54) **Kontinuierliches Verfahren zur Herstellung von Menthol in reiner oder angereicherter Form**

(71) Anmelder: BASF SE, 67056 Ludwigshafen (DE)
(72) Erfinder: Heydrich, Gunnar, Dr., 67117 Limburgerhof (DE); Gralla, Gabriele, Dr., 68161 Mannheim (DE); Ebel, Klaus, Dr., 68623 Lampertheim (DE); Krause, Wolfgang, Dr., 68782 Brühl-Rohrhof (DE); Kashani-Shirazi, Nawid, 68549 Ilvesheim (DE)

(57) **Zusammenfassung**

Die vorliegende Erfindung betrifft ein kontinuierliches Verfahren zur Herstellung von racemischem oder optisch aktivem Menthol in reiner oder angereicherter Form durch destillative Abtrennung von Menthol aus Stoffgemischen die im wesentlichen Menthol und dessen Diastereomere enthalten. Dabei wird die destillative Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

## Beschreibung

Die vorliegende Erfindung betrifft ein kontinuierliches Verfahren zur Herstellung von racemischem oder optisch aktivem Menthol, speziell von L-Menthol, in reiner oder angereicherter Form durch destillative Abtrennung von Menthol aus Stoffgemischen die im wesentlichen Menthol enthalten. Dabei wird die destillative Abtrennung in einer Trennwandkolonne vorgenommen.

Menthol, insbesondere L-Menthol stellt eine der weltweit bedeutendsten Aromachemikalien dar und wird aufgrund seiner kühlenden Eigenschaften und des frischen Pfefferminzaromas in einer Vielzahl von Produkten eingesetzt.

In der Literatur sind verschiedene Reinigungsverfahren für Menthol beschrieben. So sind dem Fachmann neben der fraktionierter Destillation mit und ohne Wasserdampf wie beispielsweise in der DE 568 085 oder DE 1 189 073 sowie der US 1,930,411, JP 27003884 oder JP 32009869 beschrieben, Extraktionsverfahren und Kristallisationsverfahren bekannt.

Diese Verfahren werden zum Teil auch in Kombination, z.B. als Kombinationen aus Kristallisation und fraktionierter Destillation oder auch in Kombination mit chemischen Umsetzungen bzw. Derivatisierungen eingesetzt.

Die GB 285,394 betrifft ein Verfahren zur Herstellung von racemischem Menthol durch Hydrierung von Thymol, fraktionierte Destillation der daraus erhaltenen Gemische und anschließendes Ausfrieren von Neomenthol aus den Mentholfraktionen.

Die GB 285,833 beschreibt ein Verfahren zur Herstellung von Thymol durch fraktionierte Destillation von Gemischen , die aus der Kondensation von Kresol mit Aceton erhalten wurden und neben Thymol isomere Methyl-isopropyl-phenole enthalten.

Die US 2,827,497 offenbart ein Verfahren bei dem durch fraktionerte Destillation und fraktionierte Kristallisation gewonnen Diastereomerngemische des Menthols einer Oxidation unterzogen werden und anschließend durch abermalige fraktionierte Destillation weiter aufgereinigt werden.

Die EP 0 242 778 beschreibt ein Verfahren zur Trennung von Diataeromerengemischen wie u.a. Gemischen von Menthol, Isomenthol, Neomenthol und Neoisomenthol durch Extraktivdestillation, d.h. durch Destillation unter Zusatz speziellerer Hilfsstoffe wie beispielsweise Bernsteinsäurediamid.

Die beschriebenen Verfahren weisen zumeist den Nachteil auf, dass Hilfsstoffe verwendet werden (Wasserdampf oder Extraktivdestillation) oder Feststoffe anfallen. Die fraktionierenden Batch-Destillationen sind zumeist nachteilig hinsichtlich ihrer Ausbeute an Wertprodukt, da das Produkt längere Zeit thermisch belastet wird.

Die EP 1 514 955 betrifft ein Verfahren zur destillativen Aufarbeitung des Elektrolyseaustrages der elektrochemischen Oxidation von 1,1,2,2-Tetramethoxyethan mit Methanol zu Trimethylorthoformiat in einem flüssigen Elektrolyten, wobei eine Trennwandkolonne mit 30 bis 150 theoretischen Trennstufen eingesetzt wird.

Die DE 103 30 934 offenbart ein Verfahren zur kontinuierlichen Isolierung von Citronellal oder Citronellol aus einem mindestens eine dieser Verbindungen enthaltenden Rohgemisch durch Rektifikation. Dabei werden vorzugsweise solche Ausgangsgemische eingesetzt, die durch partielle Hydrierung von Citral bzw. Citronellal erhalten sind.

Die DE 102 23974 betrifft ein Verfahren zur kontinuierlichen Isolierung zweier stereoisomerer Isoprenoidalkohole, speziell Nerol und Geraniol, aus einem Rohgeisch durch Rektifikation, wobei das Rohgemisch seitlich in eine Zulaufkolonne eingeführt wird, man wenigstens eine mit der Zulaufkolonne gekoppelte Abzugskolonne vorsieht und aus der Abzugskolonne einen ersten und einen zweiten Isoprenoidalkohol abzieht. Dabei werden die Zulauf- und die Abzugskolonne so gekoppelt, dass zumindest im Bereich des Abzugs der Isoprenoidalkohole keine Quervermischung von Brüden und Kondensat erfolgt.

Die destillative Reinigung des Menthols, speziell des L-Menthols von seinen Diastereomeren Neoiso- und Iso-Menthol, ist insbesondere aufgrund des sehr geringen Siedepunktsunterschiedes von ca. 2°C bei Umgebungsdruck üblicherweise sehr aufwändig.

Ausgehend von diesem Stand der Technik bestand die Aufgabe der vorliegenden Erfindung in der Bereitstellung eines Verfahrens zur Herstellung von möglichst reinem bzw. angereichertem Menthol ausgehend von Gemischen, die neben Menthol unerwünschte Diastereomere des Menthols und gegebenenfalls auch Isopulegol bzw. dessen Isomere und gegebenenfalls auch Menthone enthalten. Das Verfahren soll insbesondere geeignet sein zur Herstellung von reinem bzw. angereicherten Menthol ausgehend von Gemischen, in denen Menthol bereits als überwiegende Hauptkomponente vorliegt und welche die genannten unerwünschten Komponenten nur in sehr geringem Ausmaß bzw. als Verunreinigungen enthalten. Dies ist durch die bekannten Verfahren nur schwer und mit hohen Ausbeuteverlusten möglich. Das Verfahren soll mit geringem apparativem Aufwand, wirtschaftlich und in technischem Maßstab durchführbar sein und dabei insbesondere nur in geringem Ausmaß zur Bildung von Zersetzungs- bzw. Nebenprodukten führen, d.h. das gewünschte Produkt in hoher Reinheit und in möglichst hoher Ausbeute liefern.

Die Aufgabe wurde erfindungsgemäß gelöst durch die Bereitstellung eines kontinuierlichen Verfahrens zur Herstellung von racemischem oder optisch aktivem Menthol der Formel (I) 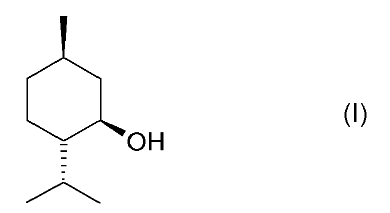 in reiner oder angereicherter Form durch destillative Abtrennung von racemischem oder optisch aktivem Menthol aus Stoffgemischen enthaltend racemisches oder optisch aktives Menthol und Diastereomere des Menthols, wobei man die destillative Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

Als Ausgangsstoffe zur Durchführung des erfindungsgemäßen Verfahrens dienen Stoffgemische, die racemisches oder optisch aktives Menthol, bevorzugt optisch aktives Menthol, besonders bevorzugt L-Menthol und Diastereomere des Menthols enthalten.

Als Diastereomere des Menthols seien die Verbindungen neo-Menthol der Formel (V), neo-Isomenthol der Formel (VI) und iso-Menthol der Formel (VII) 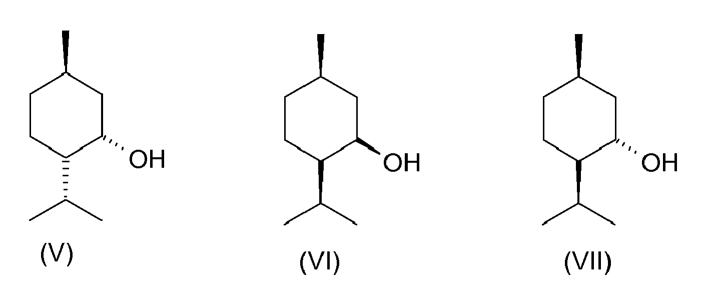 die, je nach Art des als Ausgangsstoff dienenden Gemisches in racemischer oder nicht-racemischer, d.h. optisch aktiver Form vorliegen können, genannt. Die genannten Diastereomere können in den erfindungsgemäß einzusetzenden Stoffgemischen einzeln oder in Form von Gemischen untereinander vorliegen. Die als Ausgangsstoff im Rahmen des erfindungsgemäßen Verfahrens einzusetzenden Stoffgemische enthalten neben Menthol der Formel (I) in racemischer oder optisch aktiver Form zumindest eines der Diastereomere der Formeln (V), (VI) oder (VII), üblicherweise jedoch ein Gemisch aus zwei oder allen drei der genannten Diastereomere.

Im Rahmen des erfindungsgemäßen Verfahrens können bevorzugt auch solche Stoffgemische eingesetzt werden, die neben den vorstehend genannten Diastereomeren des Menthols auch Isopulegol der Formel (II) 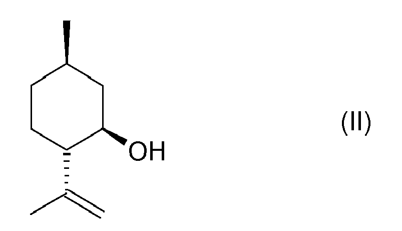 und/oder dessen Diastereomere sowie gegebenfalls Menthon der Formel (III) und/oder Isomenthon der Formel (IV) 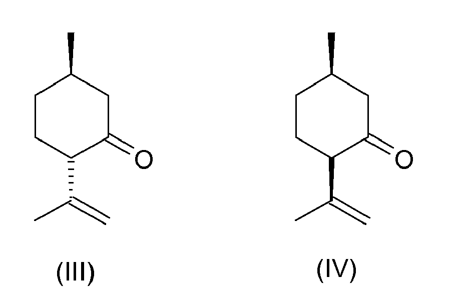 enthalten.

Die genannten Verbindungen können dabei, je nach Art, Ursprung oder Herstellverfahren des jeweils eingesetzten Stoffgemisches in racemischer oder optisch aktiver Form vorliegen.

Als Diastereomere des Isopulegols der Formel (II), insbesondere L-Isopulegol seien neo-Isopulegol der Formel (VIII), neoiso-Isopulegol der Formel (IX) und iso-Isopulegol der Formel (X), 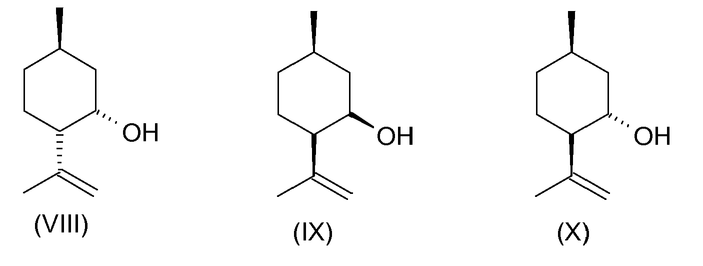 genannt, die ebenfalls, je nach Art des als Ausgangsstoff dienenden Gemisches in racemischer oder nicht-racemischer Form vorliegen können, genannt.

Eine bevorzugte Ausführungsform des erfindungsgemäßen Verfahrens betrifft die Herstellung von L-Menthol in reiner oder angereicherter Form durch destillative Abtrennung von L-Menthol aus Stoffgemischen enthaltend L-Menthol und Diastereomere des Menthols der Formeln (V), (VI) und/oder (VII) und gegebenenfalls Isopulegol der Formel (II) und/oder dessen Diastereomere der Formeln (VIII), (IX) und/oder (X) und gegebenenfalls Menthon der Formel (III) bzw. Isomenthon der Formel (IV).

Als Einsatzstoffe zur Durchführung des erfindungsgemäßen Verfahrens eignen sich Stoffgemische die racemisches oder optisch aktives Menthol, bevorzugt L-Menthol in optisch aktiver Form enthalten, bevorzugt solche, die zum überwiegenden Teil aus racemischem oder optisch aktivem Menthol, bevorzugt L-Menthol bestehen. Bevorzugt sind darunter solche Stoffgemische, die mindestens 80 Gew.-% oder besser 85 oder noch besser 90 Gew.-% bis 99,9 Gew.-%, besonders bevorzugt 95 bis 99,8 Gew.-% und ganz besonders bevorzugt mindestens 96 Gew.-%, 97 Gew.-% oder am meisten bevorzugt mindestens 98 Gew.-% bis 99,7 Gew.-%, 99,6 Gew.-% oder am meisten bevorzugt bis 99,5 Gew.-% racemisches oder optisch aktives Menthol, bevorzugt L-Menthol enthalten und daneben in geringem Ausmaß, d.h. zu einem Anteil von bis zu 20, bevorzugt von 0,1 bis zu 10 Gew.-% und besonders bevorzugt von 0,2 bis zu 5 Gew.-%, insbesondere bevorzugt von 0,3 oder besser 0,4 Gew.-% bis zu 2,5 Gew.-%, noch mehr bevorzugt bis zu 1,5 Gew.-%, besser bis zu 1 Gew.-% und am meisten bevorzugt bis zu 0,5 Gew.-% noch weitere Komponenten wie beispielsweise Diastereomere des Menthols, Nebenprodukte wie Isopulgol bzw. dessen Diastereomere oder Menthon oder Isomenthon oder sonstige Verunreinigungen wie beispielsweise Lösemittelreste oder Wasser enthalten.

Bei Einsatz von Stoffgemischen, die Menthol in optisch aktiver Form, bevorzugt L-Menthol enthalten, liegt dieses üblicherweise in einem Enantiomerenüberschuss von 90% ee oder darüber, bevorzugt 95% ee, besonders bevorzugt 97% ee oder noch mehr bevorzugt 98% ee oder darüber vor. Aus diesen Stoffgemische werden im Rahmen des erfindungsgemäßen Verfahrens entsprechend optisch aktives Menthol, bevorzugt L-Menthol in reiner oder angereicherter Form erhalten, wobei der Enantiomerenüberschuss des erhaltenen Produktes in der Regel dem Enantiomerenüberschuss des Menthols im eingesetzten Stoffgemisch zumindest weitestgehend entspricht. Bei Einsatz von racemischem Menthol enthaltenden Stoffgemischen erhält man erfindungsgemäß racemisches Menthol der Formel (I) in reiner oder angereicherter Form.

Im Rahmen einer bevorzugten Ausführungsform des erfindungsgemäßen Verfahrens dient als Ausgangsstoff ein Stoffgemisch, das einen Enantiomerenüberschuß von mehr als 99,4% ee aufweist. Im Rahmen einer weiteren bevorzugten Ausführungsform des erfindungsgemäßen Verfahrens dient als Ausgangsstoff ein Stoffgemisch, dass zu mindestens 98 Gew.-% aus Menthol (L- bzw. auch D-Menthol, bevorzugt L-Menthol) und in Summe zu bis zu 2 Gew.-% (jeweils bezogen auf das Gemisch) aus Diastereomeren des Menthols und/oder Isopulegol und dessen Diastereomere (jeweils in der d-oder I-Form) und/oder Isomenthon bzw. Menthon und/oder anderen Komponenten, wie Alkohole, Ketone, Aldehyde, Kohlenwasserstoffe oder Wasser besteht, wobei der Gehalt an Menthon und/oder Isomenthon und der Gehalt an anderen Komponenten jeweils weniger als 1 Gew.% (bezogen auf das Gemisch) beträgt.

Ein bevorzugter Einsatzstoff ist synthetisch hergestelltes Menthol, bevorzugt L-Menthol in optisch aktiver Form, insbesondere solches, das durch Hydrierung von Isopulegol bzw. L-Isopulegol hergestellt wurde. Isopulegol, speziell L-Isopulegol kann seinerseits durch Aufreinigung von synthetisch hergestelltem Isopulegol durch Kristallisation, insbesondere durch Schmelzekristallisation erhalten werden, wie beispielsweise in der DE 10 2005 040 655 beschrieben.

Weiterhin können auch Gemische von Menthol und Diasteremeren des Menthols eingesetzt werden, die auf anderen Synthesewegen hergestellt wurden, beispielsweise solche, wie sie durch die Hydrierung von Thymol erhalten werden können.

Weiterhin eignet sich handelsübliches L-Menthol aus natürlichen pflanzlichen Quellen, beispielsweise solches, wie es z.B. aus der Mentha Arvensis gewonnen wird.

Die erfindungsgemäße destillative Abtrennung wird üblicherweise so durchgeführt, dass man das eingesetzte Menthol, bevorzugt L-Menthol-enthaltende Stoffgemisch in jeweils eine oder mehrere Leichtsieder-, Mittelsieder- und Schwersiederfraktion bzw. - fraktionen auftrennt und Menthol, bevorzugt L-Menthol in reiner oder angereicherter Form als Mittelsiederfraktion an der Seitenabzugsstelle der eingesetzten Trennwandkolonne in flüssiger oder gasförmiger Form entnimmt.

Bei dem erfindungsgemäßen Verfahren handelt es sich demnach auch um ein kontinuierliches Verfahren zur Isolierung von Menthol, bevorzugt von L-Menthol, vorzugsweise um ein kontinuierliches Verfahren zur Isolierung von Menthol in reiner oder angereicherter Form durch destillative Abtrennung von Menthol aus Stoffgemischen enthaltend Menthol und seine wie vorstehend beschriebenen Diastereomere, wobei man die destillative Abtrennung in einer Trennwandkolonne mit 80 bis 200 theoretischen Trennstufen und einer oder mehrerer Seitenabzugsstellen bei einem absoluten Betriebsdruck von 5 bis 500 mbar durchführt.

Die erfindungsgemäß einzusetzende Trennwandkolonne weist eine Gesamtstufenzahl von 50 bis 300, bevorzugt 100 bis 200 und ganz besonders bevorzugt 120 bis 180 theoretische Trennstufen und eine oder mehrere, bevorzugt 1 bis 3, insbesondere 1 oder 2 und ganz besonders bevorzugt 1 Seitenabzugsstelle bzw. Seitenabzugsstellen auf.

Das erfindungsgemäße Verfahren wird bei einem absoluten Betriebsdruck in der Trennwandkolonne von 5 bis 500 mbar, bevorzugt von 10 bis 200 mbar, besonders bevorzugt von 20 bis 120 mbar und ganz besonders bevorzugt von 20 bis 100 mbar und insbesondere bevorzugt bei einem absoluten Betriebsdruck von 40 bis 100 mbar durchgeführt. Bevorzugt betreibt man dabei die Trennwandkolonne so, dass der absolute Kopfdruck 10 bis 100 mbar, besonders bevorzugt 30 bis 80 mbar,ganz besonders bevorzugt 20 bis 60 mbar und insbesondere bevorzugt 40 bis 60 mbar beträgt. Ebenfalls bevorzugt betreibt man dabei die Trennwandkolonne so, dass der absolute Sumpfdruck 20 bis 500 mbar, besonders bevorzugt 40 bis 200 mbar und ganz besonders bevorzugt 50 bis 100 mbar beträgt.

Das Rücklaufverhältnis kann bei der Durchführung des erfindungsgemäßen Verfahrens in breiten Grenzen variiert werden und liegt üblicherweise bei etwa 5 zu 1 bis etwa 2000 zu 1, bevorzugt etwa 20 zu 1 bis 1000 zu 1 und besonders bevorzugt bei etwa 50 zu 1 bis etwa 500 zu 1. Vorteilhaft ist auch eine Dephlegmator-Fahrweise, d.h. es wird im Kopfkondensator der Kolonne nur der Rücklauf kondensiert und wieder der Kolonne zugeführt. In einem solchen energetisch günstigen Fall der Teilkondensation fällt das auszuschleusende Kopfprodukt ausschließlich im Nachkühler an, der bei niedrigerer Temperatur betrieben werden kann. Dabei ist es vorteilhaft, einen Wärmeträgerkreislauf so vorzusehen, dass die Temperatur des Kühlmediums im Nachkühler in einem Bereich von 5°C bis von etwa 50°C temperiert werden kann, um gegebenenfalls durch Desublimation gebildete Feststoffe von Zeit zu Zeit wieder aufschmelzen zu können.

Aus diesem Grund ist es auch von Vorteil, eine Möglichkeit vorzusehen, den Hauptkondensator und/oder den Nachkondensator der Kolonne mit einem von 0°C bis 60°C, bevorzugt von 20 bis 60°C temperierbaren Wärmeträgermedium (Kühlmedium) zu speisen. Zu diesem Zweck kann beispielsweise Wasser mit Hilfe einer Kreiselpumpe über den Wärmetauscher umgepumpt werden und über eine Temperaturregelung je nach Bedarf kaltes oder warmes Wasser in diesen Umpumpkreis eingespeist werden. Selbstverständlich ist auch eine elektrische Beheizung dieses Kreises mit einem in den Kreislauf eingebauten Durchlauferhitzer oder eine konventionelle Beheizung mit Dampf möglich.

Durch das erfindungsgemäße Verfahren ist Menthol, vorzugsweise L-Menthol in reiner oder angereicherter Form zugänglich. Unter dem Begriff Menthol in angereicherter Form sind Menthol, bevorzugt L-Menthol-haltige Stoffgemische zu verstehen, die einen höheren Gehalt an Menthol bzw. L-Menthol aufweisen als das jeweils erfindungsgemäß eingesetzte Menthol bzw. bevorzugt L-Menthol enthaltende Stoffgemisch. Bevorzugt ist unter dem Begriff Menthol in angereicherter Form solches Menthol, bevorzugt L-Menthol zu verstehen, dass eine Reinheit, d.h. einen -Gehalt von mehr als 80 bis 99,5 Gew.-%, bevorzugt von 85 bis 99,5 Gew.-%, besonders bevorzugt von 90 oder noch mehr bevorzugt von 95 Gew.-% bis 99,5 Gew.-% aufweist. Das erfindungsgemäße Verfahren ermöglicht auch die Herstellung von Menthol, bevorzugt L-Menthol) in reiner Form. Unter dem Begriff Menthol in reiner Form ist Menthol, bevorzugt L-Menthol mit einem Gehalt von größer oder gleich 99 Gew.-%, bevorzugt größer oder gleich 99,1 Gew.-%, bevorzugt von mindestens 99,2 Gew.-%, weiter bevorzugt von mindestens 99,3 Gew.-%, noch mehr bevorzugt von mindestens 99,4 Gew.-% und insbesondere bevorzugt von mindestens 99,5 Gew.-% bis, wiederum bevorzugt von mindestens 99,6 Gew.-%, weiterhin bevorzugt von mindestens 99,7 Gew.-% und am meisten bevorzugt von 99,8 Gew.-% bis 99,99 Gew.-%, bevorzugt bis 99,98 Gew.-%, besonders bevorzugt bis 99,97 Gew.-%, noch mehr bevorzugt bis 99,96 und am meisten bevorzugt bis 99,95 Gew.% zu verstehen. Dabei beziehen sich die Angeben in Gew.-% wie alle Angaben in Gew.-% im Rahmen der vorliegenden Erfindung auf die Gesamtmenge des jeweiligen Gemisches.

Der Zulauf, d.h. das einzusetzende Stoffgemisch kann flüssig oder gasförmig in die Trennwandkolonne geführt und dort in eine Kopf- und Sumpffraktion sowie einen oder mehrere Seitenabläufe, bevorzugt in einen Seitenablauf aufgetrennt werden. In einem Seitenablauf fällt das Wertprodukt Menthol, bevorzugt L-Menthol in der gewünschten Reinheit, d.h. in angereicherter oder reiner Form an. In einer besonderen Ausführungsform des erfindungsgemäßen Verfahrens ist dem Kopfkondensator der Kolonne ein Nachkondensator nachgeschaltet, der, wie oben ausgeführt, mit einer im Temperaturbereich von 0 bis 60°C, bevorzugt von 20 bis 60°C temperierbarer Kühlflüssigkeit (beispielsweise mit glykolhaltigem Wasser) gekühlt ist und in dem noch eine Menthol-arme Leichtsiederfraktion anfällt.

Für die kontinuierliche destillative Zerlegung von Mehrstoffgemischen sind nach dem Stand der Technik verschiedene Verfahrensvarianten gebräuchlich. Im einfachsten Fall wird das Zulaufgemisch in zwei Fraktionen, eine leichtsiedende Kopffraktion und eine schwersiedende Sumpffraktion, zerlegt. Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Die Anwendungsmöglichkeit von Destillationskolonnen mit Seitenabzügen ist jedoch dadurch stark eingeschränkt, dass nach dem Stand der Technik die an den Seitenabzugsstellen entnommenen Produkte nie völlig rein sind. Bei Seitenentnahmen im Verstärkungsteil, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil, die meist dampfförmig erfolgen, bei denen das Seitenprodukt noch Hochsiederanteile aufweist. Die Verwendung von konventionellen Seitenabzugskolonnen ist daher auf Fälle begrenzt, in denen verunreinigte Seitenprodukte zulässig sind.

Eine Abhilfemöglichkeit bieten Trennwandkolonnen. Dieser Kolonnentyp ist beispielsweise beschrieben in US 2,471,134; US 4,230,533; EP 0 122 367; EP 0 126 288; EP 0 133 510; Chem. Eng. Technol. 10 (1987) 92 - 98; Chem.-Ing.-Tech. 61 (1989) Nr.1, 16 - 25; Gas Separation and Purification 4 (1990) 109 - 114; Process Engineering 2 (1993) 33 - 34; Trans IChemE 72 (1994) Part A 639 - 644 und Chemical Engineering 7 (1997) 72 - 76.

Bei dieser Bauart ist es möglich, Seitenprodukte ebenfalls in reiner Form zu entnehmen. Im mittleren Bereich oberhalb und unterhalb der Zulaufstelle und der Seitenentnahme ist eine Trennwand angebracht, die den Zulaufteil gegenüber dem Entnahmeteil abdichtet und in diesem Kolonnenteil eine Quervermischung von Flüssigkeits- und Brüdenströmen unterbindet. Hierdurch verringert sich bei der Auftrennung von Vielstoffgemischen die Zahl der insgesamt benötigten Destillationskolonnen. Da dieser Kolonnentyp eine apparative Vereinfachung von thermisch gekoppelten Destillationskolonnen darstellt, weist er darüber hinaus auch einen besonders niedrigen Energieverbrauch auf. Eine Beschreibung von thermisch gekoppelten Destillationskolonnen, die in verschiedener apparativer Ausgestaltung ausgeführt sein können, findet sich ebenfalls in den oben genannten Stellen in der Fachliteratur. Trennwandkolonnen und thermisch gekoppelte Kolonnen bieten gegenüber der Anordnung von konventionellen Destillationskolonnen sowohl hinsichtlich des Energiebedarfs als auch der Investitionskosten Vorteile und werden daher zunehmend industriell eingesetzt.

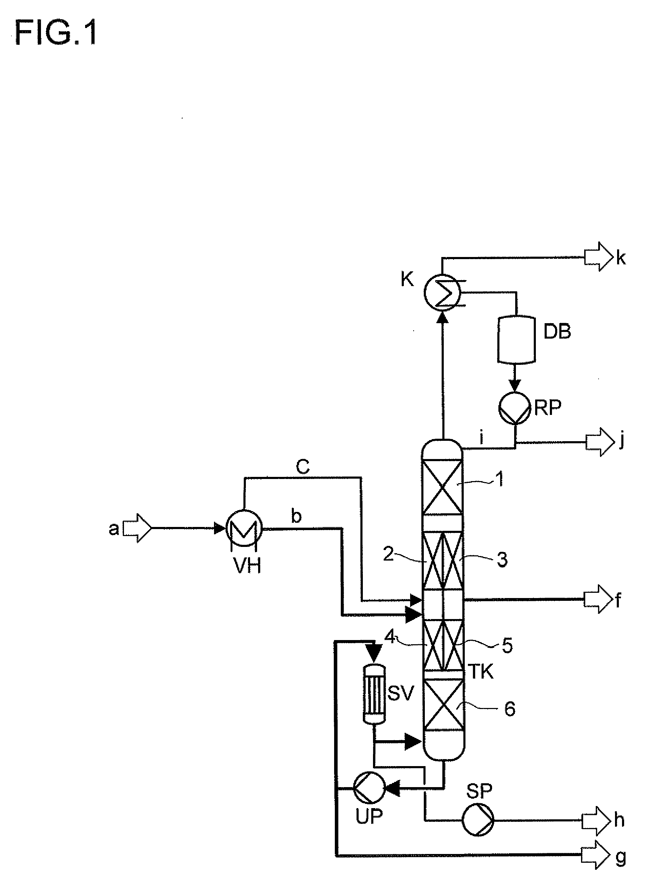

Figur 1 zeigt eine bevorzugte Ausführungsform der erfindungsgemäßen Auftrennung des einzusetzenden Menthol enthaltenden Stoffgemisches in eine Menthol-arme Kopffraktion (j), eine Menthol-reiche Seitenfraktion (f) und eine Sumpffraktion (g). Der Menthol-haltige Zulauf zur Trennwandkolonne kann flüssig (b) gasförmig (c), bzw. gasförmig und flüssig erfolgen.

Das erfindungsgemäße Verfahren wird kontinuierlich durchgeführt. Demzufolge werden die als Ausgangsstoff einzusetzenden Menthol, bevorzugt L-Menthol enthaltenden Stoffgemische der Trennwandkolonne kontinuierlich zugeführt und die erfindungsgemäß erhaltenen Produkte (Fraktionen) bzw. Nebenprodukte kontinuierlich ausgetragen.

Der Kolonne wird üblicherweise ein weiterer Kondensator nachgeschaltet, dessen Arbeitstemperatur 10 bis 40 K, bevorzugt 20 bis 30 K unter der Arbeitstemperatur des Kopfkondensators der Trennwandkolonne liegt. Mit dessen Hilfe kann ein Großteil der noch im Kopfstrom (k) enthaltenen Leichtsieder niedergeschlagen werden.

Darüber hinaus kann es sowohl bei Trennwandkolonnen von Vorteil sein, den Zulaufstrom einer Vorverdampfung zu unterziehen und anschließend zweiphasig oder in Form von zwei Strömen der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Die erfindungsgemäß einzusetzenden Trennwandkolonnen können sowohl als Packungskolonnen mit Füllkörpern oder geordneten Packungen oder als Bodenkolonnen ausgeführt werden. Bei dem erfindungsgemäßen Verfahren zur Herstellung von Menthol in reiner oder angereicherter Form empfiehlt es sich, Packungskolonnen einzusetzen. Dabei sind geordnete Blech- oder Gewebepackungen mit einer spezifischen Oberfläche von etwa 100 bis 750 m²/m³, bevorzugt etwa 350 bis 500 m²/m³ besonders geeignet.

Falls, wie im Fall der vorliegenden Erfindung, besonders hohe Anforderungen an die Reinheiten der Produkte gestellt werden, ist es günstig, die Trennwand mit einer thermischen Isolierung auszustatten. Eine Beschreibung der verschiedenen Möglichkeiten der thermischen Isolierung der Trennwand findet sich in EP-A 0 640 367. Eine doppelwandige Ausführung mit einem zwischen liegenden engen Gasraum ist besonders günstig.

Für die Regelung von Trennwandkolonnen und thermisch gekoppelten Kolonne wurden verschiedene Regelungsstrategien beschrieben. Beschreibungen finden sich in US 4,230,533; DE 35 22 234 ; EP 0 780 147; Process Engineering 2 (1993) 33 - 34 und Ind. Eng. Chem. Res. 34 (1995), 2094 - 2103.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert. Diese Schlüsselkomponenten sind im Rahmen der vorliegenden Erfindung Iso-Menthol als hochsiedende Nebenkomponente und Neo-Menthol bzw. ein Gemisch von Neo-und Neosio-Menthol als tiefsiedende Nebenkomponente.

Die Einhaltung der Spezifikation für die Hochsieder in der Mittelsiederfraktion kann beispielsweise über das Aufteilungsverhältnis der Flüssigkeit am oberen Ende der Trennwand geregelt. Dabei wird das Aufteilungsverhältnis der Flüssigkeit am oberen Ende der Trennwand vorzugsweise so eingestellt, dass die Konzentration der Schlüsselkomponenten für die Hochsiederfraktion in der Flüssigkeit am oberen Ende der Trennwand 10 bis 80%, bevorzugt 30 bis 50%, des Wertes ausmacht, der im Seitenabzugsprodukt erzielt werden soll. Die Flüssigkeitsaufteilung wird vorzugsweise dahingehend eingestellt, dass bei höheren Gehalten an Schlüsselkomponenten der Hochsiederfraktion mehr und bei niedrigeren Gehalten an Schlüsselkomponenten der Hochsiederfraktion weniger Flüssigkeit auf den Zulaufteil geleitet wird.

Entsprechend kann die Spezifikation für die Leichtsieder in der Mittelsiederfraktion durch die Heizleistung geregelt werden. Hierbei wird beispielsweise die Heizleistung im Verdampfer so eingestellt, dass die Konzentration an Schlüsselkomponenten der Leichtsiederfraktion in der Flüssigkeit am unteren Ende der Trennwand 10 bis 80, bevorzugt 30 bis 50% des Wertes ausmacht, der im Seitenabzugsprodukt erzielt werden soll, und die Heizleistung wird vorzugsweise dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Zur Kompensation von Störungen der Zulaufmenge oder der Zulaufkonzentration erwies es sich zudem als vorteilhaft, durch einen entsprechenden Regelmechanismus beispielsweise durch geeignete Regelvorschriften im Prozessleitsystem sicherzustellen, dass die Mengenströme der Flüssigkeiten, die auf die Kolonnenteile (2), d.h. den Verstärkungsteil des Zulaufteils, und (5), d.h. den Abtriebsteil des Entnahmeteils, nicht unter 30 % ihres Normalwertes sinken können.

Zur Entnahme und Aufteilung der Flüssigkeiten am oberen Ende der Trennwand und an der Seitenentnahmestelle eignen sich sowohl innenliegende als auch außerhalb der Kolonne angeordnete Auffangräume für die Flüssigkeit, welche die Funktion einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglichen. Bei der Verwendung von gepackten Kolonnen wird die Flüssigkeit zunächst in Sammlern gefasst und von dort aus in einen innenliegenden oder außenliegenden Auffangraum geleitet.

Im Rahmen einer besonders bevorzugten Ausführungsform führt man das erfindungsgemäße Verfahren in einer Anlage durch, wie sie schematisch in Figur 1 gezeigt ist. Die bevorzugte Ausführungsform zeichnet sich dadurch aus, dass man eine Trennwandkolonne (TK) einsetzt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist.

Das als Einsatzstoff dienende Menthol enthaltende Stoffgemisch (a) wird erfindungsgemäß vorzugsweise in den mittleren Bereich des Zulaufteils (2, 4) zugeführt, das Menthol, bevorzugt L-Menthol in reiner oder angereicherter Form als Seitenabzug aus dem mittleren Bereich des Entnahmeteils (3, 5) gewonnen und eine oder mehrere Leichtsiederfraktionen aus dem oberen gemeinsamen Kolonnenbereich (1) und eine oder mehrere Hochsiederfraktionen aus dem unteren gemeinsamen Kolonnenbereich (6) abgeführt.

Der Zulaufstrom (a) kann über einen Vorheizer (VH) als flüssiger (b), gasförmiger (c) oder teilweise flüssig und gasförmiger Strom in die Kolonne (TK) eingeleitet werden. Der Kopfstrom der Kolonne wird im Kondensator (K) ganz oder teilweise kondensiert. Bei einer teilweisen Kondensation (Dephlegmator-Betrieb) enthält der Abgasstrom (k) des Kopfkondensators (K) üblicherweise noch merkliche Mengen an kondensierbaren Leichtsiedern, die dann in einem bei niedriger Temperatur betriebenen Nachkondensator niedergeschlagen werden können.

Der Kopfkondensator (K) und/oder der Nachkondensator können beispielsweise als Plattenapparat ausgeführt und in den Kolonnenmantel, bevorzugt in den Kolonnenkopf integriert sein. Zur Vermeidung von Feststoffbildung kann es von Vorteil sein, den Kondensator der Kolonne zu temperieren, beispielsweise auf Temperaturen von etwa 30 bis etwa 50°C.

Das im Kondensator (K) niedergeschlagene Kopfprodukt wird in dem Destillatbehälter (DB) gepuffert und über die Rücklaufpumpe (RP) als Kolonnenrücklauf (i) der Kolonne wieder zugeführt. Bei Bedarf lässt sich hieraus auch eine Destillatfraktion (j) gewinnen. Bei Integration des Kondensators in den Kolonnenkopf kann auf den Destillatbehälter (DB) und die Rücklaufpumpe verzichtet (RP) werden.

Der Sumpfstrom wird vorteilhaft über die Umlaufpumpe (UP) dem Sumpfverdampfer (SV) zugeführt, der bevorzugt als Fallfilmverdampfer ausgeführt ist. Diesem Umpumpstrom kann auch der Sumpfaustrag (g) der Kolonne (TK) entnommen werden. Vorteilhaft wird der Sumpfstrom (Hochsiederfraktion) der Kolonne als flüssiger Strom (h) hinter dem Sumpfverdampfer, ggf. mit Hilfe einer kleineren Pumpe (SP) entnommen.

Als Sumpfverdampfer für die Trennwandkolonne kann vorteilhaft ein Dünnschichtapparat, beispielsweise ein Fallfilmverdampfer, verwendet werden.

Das Wertprodukt wird als flüssiger Seitenabzug, Strom (f), aus dem Entnahmeteil der Trennwandkolonne (TK) abgezogen. Es ist auch möglich, bei Bedarf den Wertprodukt-Strom (f) als gasförmigen Abzug zu entnehmen, dann wird aber üblicherweise ein weiterer Kondensator benötigt. Aufgrund des Festpunktes von L-Menthol in reiner oder angereicherter Form zwischen 41 und 44°C ist es vorteilhaft, alle produktführenden Apparate (neben der Kolonne auch alle Behälter und Pumpen) und Leitungen sowie bevorzugt alle Apparate und Leitungen des Vakuumsystems zu isolieren, d.h. thermisch mit geeigneten Materialien zu isolieren und mit Begleitheizungen zu versehen. Dabei sind z.B. den Rohren beigelegte elektrische Heizleitungen, die mit geeigneten Geräten auf Temperaturen von bis zu 70°C, bevorzugt von 45 bis 70°C, noch mehr bevorzugt auf Temperaturen bis zu 60°C, insbesondere bevorzugt von 45 bis 60°C geregelt werden, vorteilhaft. Alternativ können auch konventionelle Begleitheizungssysteme wie z.B. durch mit Warmwasser durchströmte Doppelmantelrohre zum Einsatz kommen.

Der obere gemeinsame Teilbereich (1) der Kolonne weist üblicherweise 5 bis 50%, der Verstärkungsteil (2) des Zulaufteils der Kolonne 5 bis 50%, der Abtriebsteil (4) des Zulaufteils der Kolonne 2 bis 50%, der Abtriebsteil (2) des Entnahmeteils der Kolonne 5 bis 50%, der Verstärkungsteil (5) des Entnahmeteils 2 bis 50%, und der gemeinsame untere Teil (6) der Kolonne 5 bis 50% der Gesamtzahl der theoretischen Trennstufen der Kolonne auf, wobei sich die gewählten Prozentangaben zu 100% addieren müssen.

Bevorzugt weist der obere gemeinsame Teilbereich (1) der Kolonne 10 bis 25%, der Verstärkungsteil (2) des Zulaufteils der Kolonne 15 bis 30%, der Abtriebsteil (4) des Zulaufteils der Kolonne 15 bis 30%, der Abtriebsteil (2) des Entnahmeteils der Kolonne 15 bis 30%, der Verstärkungsteil (5) des Entnahmeteils 15 bis 30%, und der gemeinsame untere Teil (6) der Kolonne 10 bis 25% der Gesamtzahl der theoretischen Trennstufen der Kolonne auf, wobei sich die gewählten Prozentangaben zu 100% addieren müssen.

Die Summe der Zahl der theoretischen Trennstufen der Teilbereiche (2) und (4) im Zulaufteil beträgt bevorzugt 80 bis 110%, besonders bevorzugt 95 bis 105% der Summe der Zahl der Trennstufen der Teilbereiche (3) und (5) im Entnahmeteil.

Im Rahmen einer bevorzugten Ausführungsform des erfindungsgemäßen Verfahrens sind die Zulaufstelle und die Seitenabzugsstelle hinsichtlich der Lage der theoretischen Trennstufen auf unterschiedlicher Höhe in der Kolonne angeordnet, indem die Zulaufstelle um 1 bis 40, bevorzugt um 5 bis 20 theoretische Trennstufen höher oder niedriger anordnet als die Seitenabzugsstelle.

Es hat sich darüber hinaus als vorteilhaft erwiesen wenn der durch die Trennwand unterteilte Teilbereich der Kolonne bestehend aus den Teilbereichen (2), (3), (4) und (5) oder Teilen davon mit geordneten Packungen oder Füllkörpern (beispielsweise Gewebepackungen wie Montz A3-500 , Sulzer BX oder CY oder Blechpackungen wie Montz B1-500 (Fa. Montz) oder Mellapak (Fa. Sulzer) bestückt ist.

Der Brüdenstrom am unteren Ende der Trennwand kann durch die Wahl und/oder Dimensionierung der Trenneinbauten und/oder den Einbau druckverlusterzeugender Vorrichtungen, beispielsweise von Blenden, so eingestellt wird, dass das Verhältnis des Brüdenstroms im Zulaufteil zu dem des Entnahmeteils 0,8 bis 1,2, bevorzugt 0,9 bis 1,1 beträgt.

Die aus dem oberen gemeinsamen Teil (1) der Kolonne ablaufende Flüssigkeit wird vorteilhaft in einem in der Kolonne oder außerhalb der Kolonne angeordneten Auffangraum gesammelt und gezielt durch eine Festeinstellung oder Regelung am oberen Ende der Trennwand so aufgeteilt, dass das Verhältnis des Flüssigkeitsstroms zum Zulaufteil zu dem zum Entnahmeteil 0,1 bis 2,0 bei größtenteils flüssigen Zulauf und 1,0 bis 2 bei gasförmigem Zulauf beträgt. Dabei ist der flüssige Zulauf erfindungsgemäß bevorzugt.

Die aus dem oberen gemeinsamen Teilbereich (1) auf den Zulaufteil ablaufende Flüssigkeit kann über eine Pumpe gefördert oder über eine statische Zulaufhöhe von mindestens 1 m mengengeregelt aufgegeben werden, bevorzugt über eine Kaskadenregelung in Verbindung mit der Flüssigkeitsstandregelung des Auffangraums. Die Regelung wird bevorzugt so eingestellt, dass die auf den Zulaufteil aufgegebene Flüssigkeitsmenge nicht unter 30 % des gewünschten Normalwertes sinken kann. Darüber hinaus wird die Aufteilung der aus dem Teilbereich (3) im Entnahmeteil der Kolonne ablaufenden Flüssigkeit auf den Seitenabzug und auf den Teilbereich (5) im Entnahmeteil der Kolonne durch eine Regelung vorteilhaft so eingestellt, dass die auf den Teilbereich (5) aufgegebene Flüssigkeitsmenge nicht unter eine Höhe von 30% des gewünschten Normalwertes sinken kann. Als Normalwert sind dabei vorteilhaft die zwei- bis vierfache Menge, bezogen auf die Zulaufmenge anzunehmen.

Die Trennwandkolonne weist vorzugsweise am oberen und unteren Ende der Trennwand Probenahmemöglichkeiten auf, aus der Kolonne kontinuierlich oder in zeitlichen Abständen flüssig oder gasförmig Proben entnommen und hinsichtlich ihrer Zusammensetzung, bevorzugt gaschromatographisch, untersucht werden können.

Das Aufteilungsverhältnis der Flüssigkeit am oberen Ende der Trennwand wird vorzugsweise so eingestellt, dass die Konzentration an denjenigen Komponenten der Hochsiederfraktion, für die im Seitenabzug ein bestimmter Grenzwert für die Konzentration erzielt werden soll (speziell Iso-Menthol), in der Flüssigkeit am oberen Ende der Trennwand 10 bis 80% des Wertes ausmacht, der im Seitenabzugsprodukt erzielt werden soll. Die Flüssigkeitsaufteilung sollte bevorzugt dahingehend eingestellt werden, dass bei höheren Gehalten an Komponenten der Hochsiederfraktion mehr und bei niedrigeren Gehalten an Komponenten der Hochsiederfraktion weniger Flüssigkeit auf den Zulaufteil geleitet wird.

Die Heizleistung im Verdampfer (SV) stellt man bevorzugt so ein, dass die Konzentration an denjenigen Komponenten der Leichtsiederfraktion, für die im Seitenabzug ein bestimmter Grenzwert für die Konzentration erzielt werden soll (speziell Neoiso-Menthol), am unteren Ende der Trennwand so ein, dass die Konzentration an Komponenten der Leichtsiederfraktion in der Flüssigkeit am unteren Ende der Trennwand 10 bis 80% des Wertes ausmacht, der im Seitenabzugsprodukt erzielt werden soll. Die Heizleistung stellt man vorteilhaft dahingehend ein, dass bei höherem Gehalt an Komponenten der Leichtsiederfraktion die Heizleistung erhöht und bei niedrigerem Gehalt an Komponenten der Leichtsiederfraktion die Heizleistung verringert wird.

Die Destillatentnahme, d.h. die Entnahme der tiefsiedenden Nebenprodukte erfolgt bevorzugt temperaturgeregelt oder aber mengengeregelt, in Abhängigkeit der Menge der im Zulaufgemisch vorhandenen abzutrennenden leichtersiedenden Nebenkomponenten. Als Regeltemperatur verwendet man mit Vorteil eine Messstelle im Teilbereich (1) der Kolonne, die um 3 bis 10, bevorzugt 4 bis 6 theoretische Trennstufen unterhalb des oberen Endes der Kolonne angeordnet ist.

Die Entnahme des Sumpfprodukts erfolgt bevorzugt Temperaturgeregelt oder aber Mengengeregelt, in Abhängigkeit von der Zulaufmenge.

Die Entnahme des als Seitenprodukt gewonnenen Verfahrensproduktes Menthol, bevorzugt L-Menthol in reiner oder angereicherter Form erfolgt vorzugsweise standgeregelt wobei als Regelgröße vorzugsweise der Flüssigkeitsstand im Kolonnensumpf verwendet wird.

Der Zulaufstrom des erfindungsgemäß einzusetzenden Menthol-haltigen Stoffgemisches wird vorzugsweise teilweise oder vollständig vorverdampft und der Kolonne zweiphasig oder in Form eines gasförmigen und eines flüssigen Stromes zugeführt.

Im Rahmen einer bevorzugten Ausführungsform setzt man im Rahmen des erfindungsgemäßen Verfahrens eine Trennwandkolonne ein, deren Trennwand nicht in die Kolonne eingeschweißt ist, sondern in Form von lose gesteckten und adäquat abgedichteten Teilsegmenten gestaltet ist.

Die Flüssigkeitsverteilung in den einzelnen Teilbereichen der Kolonne kann bevorzugt gezielt ungleichmäßig eingestellt werden, wobei insbesondere in den Teilbereichen (2) und (5) die Flüssigkeit verstärkt im Wandbereich aufgegeben wird und in den Teilbereichen (3) und (4) die Flüssigkeit reduziert im Wandbereich aufgegeben wird.

Verteilungsverhältnis der rücklaufenden Flüssigkeit zwischen Abnahme- und Zulaufseite der Trennwand beträgt vorzugsweise etwa 1 zu 1 bis etwa 3 zu 1 bevorzugt etwa 1 zu 1 bis etwa 2 zu 1.

Die Lage der Trennwand in den einzelnen Teilbereichen der Kolonne kann vorteilhaft so angepasst werden, dass die Querschnitte von Zulauf- und Entnahmeteil verschiedene Flächen aufweisen.

Das erfindungsgemäß zugängliche L-Menthol in reiner oder angereicherter Form wird über den Seitenabzug, bzw. im Fall, dass weitere Seitenabzüge vorgesehen sind, über den mittleren Seitenabzug (f) kontinuierlich gewonnen und weist im Rahmen einer bevorzugten Ausführungsform einen Menthol-Gehalt von über 99,5 Gew.-%, bevorzugt von 99,5 bis 99,95 Gew.-%, einen Gehalt der anderen, wie vorstehend beschriebenen Diastereomere des Menthols von bis zu 0,3 Gew.-%, gegebenenfalls neben geringsten Mengen weiterer Verunreinigungen, auf.

Im Rahmen einer weiteren bevorzugten Ausführungsform weist das erfindungsgemäß erhaltene Menthol, bevorzugt L-Menthol in reiner oder angereicherter, bevorzugt in reiner Form einen Gehalt an Isopulegol und dessen wie vorstehend beschriebenen Diasteromeren von zusammen bis zu 0,5 Gew.-%, bevorzugt bis zu 0,3 und besonders bevorzugt bis zu 0,1 Gew.-% (bezogen auf das erhaltene Produkt) auf. Im Rahmen einer weiteren bevorzugten Ausführungsform weist das erfindungsgemäß erhaltene Menthol, bevorzugt L-Menthol in reiner oder angereicherter, bevorzugt in reiner Form einen Gehalt an Menthon und Isomenthon von bis zu 0,5 Gew.-%, bevorzugt bis zu 0,3 und besonders bevorzugt bis zu 0,1 Gew.-% (bezogen auf das erhaltene Produkt) auf.

Die folgenden Beispiele dienen der Erläuterung der Erfindung, ohne sie in irgendeiner Weise zu beschränken:

### Beispiel 1:

Eine Labor-Trennwandkolonne wurde aus fünf je 1,2 m langen Glasschüssen mit 64 mm Innendurchmesser aufgebaut. In die drei mittleren Schüsse wurde eine Trennwand aus Metallblech gesteckt. Unterhalb und oberhalb des Trennwandbereiches wurden Laborpackungen (Sulzer CY) und im Trennwandbereich Metall-Geweberinge aus Edelstahl mit 5 mm Durchmesser eingebaut. Bei Trennleistungsmessungen, die mit dem Xylol-Isomerengemischen bei einem Kopfdruck von 60 mbar durchgeführt wurden, konnte eine Gesamttrennleistung von 100 theoretischen Stufen über die gesamte Kolonne und etwa 55 theoretischen Stufen im Trennwandbereich gemessen werden. Die Gesamtzahl der vorhandenen theoretischen Stufen betrug also etwa 155. Die Kolonne wurde mit einem ölbeheizten Dünnschichtverdampfer (0,1 m²) und einem mit Kühlwasser gekühlten Kondensator ausgerüstet.

Temperaturen an verschiedenen Höhen in der Kolonne sowie der Kopfdruck und der Druckverlust über die Kolonne wurden mittels eines Messwerterfassungssystem gemessen. Die Kolonne verfügte über Durchflussmessungen in den Zu- und Abläufen sowie eine Mengenmessung des Rücklaufs, die als Regelgröße für die Vorlauftemperatur des Ölthermostaten diente. Durch diese Regelung wurde eine konstante Rücklaufmenge gewährleistet, wodurch sich auch ein konstanter Differenzdruck einstellte. Die Aufteilung der Flüssigkeitsmenge oberhalb der Trennwand auf Zulauf- und Entnahmeteil der Trennwand wurde über einen zeitlich getakteten Schwenktrichter realisiert.

Der Kolonne wurde in der Kolonnenmitte einer Höhe von 331 cm zum Zulaufteil der Trennwand 1000 g/h eines auf 90°C vorgewärmten flüssigen Menthols pflanzlicher Herkunft, das 99,58 GC-Flächen-% Menthol, 0,22 GC-Flächen-% Isopulegol, 0,11 GC-Flächen-% Neomenthol und 0,03 GC-Flächen-% Isomenthol sowie 0,02 GC-Flächen-% Neoisomenthol enthielt zugeführt. Die Kolonne wurde bei 50 mbar Kopfdruck und einem Rücklauf von 3,0 kg/h betrieben. Dabei stellte sich ein Druckverlust von etwa 34 mbar (± 1 mbar) ein. Am Kopf der Kolonne wurde eine Temperatur von 121 °C und im Sumpf eine Temperatur von 135 °C (± 0,5 K) gemessen. Der Sumpfabzug wurde über eine Waagensteuerung fest auf 2 g/h (± 1 g/h) und die Destillatabnahme auf 4 g/h (± 1 g/h) eingestellt. Das Rücklaufverhältnis betrug somit etwa 750: 1. Der Kondensator der Kolonne wurde auf 25°C temperiert, um Feststoffbildung zu vermeiden.

Die Flüssigkeit wurde oberhalb der Trennwand in einem Verhältnis von 1 : 1 (Zulauf- : Entnahmeteil) aufgeteilt. In einer Höhe von 300 cm im Abnahmeteil der Trennwand wurde ein gasförmiger Seitenabzug (f) entnommen und in einem Glaskühler kondensiert, aus dem in Abhängigkeit vom Sumpffüllstand etwa 992 bis 995 g/h Reinprodukt über eine Pumpe abgezogen wurde.

Die gewonnenen Fraktionen wurden gaschromatographisch mit Hilfe eines Standard-GC analysiert. Probenvorbereitung: Die (fest gewordene) Probe wurde unter Schmelzen auf etwa 50°C erwärmt und in Toluol gelöst. Die toluolische Lösung wurde in den Gaschromatographen eingespritzt, bei der Integration wurde entsprechend der Toluol-Peak ausgeblendet.

Gaschromatographische Analysen wurden nach der folgenden Methode durchgeführt: 50 m CP-Wax 52 CB , ID.: 0,32 mm, FD.: 1,2 µm; Injektor: 200°C; Detektor: 250 °C; 80°C - 3 °C/min auf 200 °C, - 10 °C/min auf 230 °C /15 min; t_{R} (Isopulegol): 30,07 min; t_{R} (Neo-Menthol): 31,08 min; t_{R} (Neoiso-Menthol): 32,5 min; t_{R} (Menthol): 32,8 min; t_{R} (Iso-Menthol): 33,8 min

Das am Seitenabzug gewonnene Reinprodukt enthielt neben 99,94 GC-Flächen-% L-Menthol auch 0,02 GC-Flächen-% Isomenthol und Spuren anderer Menthol-Diastereomere. Im Sumpfabzug wurden durch GC-Analyse 96,12 GC-Flächen-% L-Menthol bestimmt, das Destillat enthielt 44,7 GC-Flächen-% L-Menthol, 33,9 GC-Flächen-% Isopulegol, 12,9 GC-Flächen-% Neo-Menthol und 2,02 GC-Flächen-% Neoisomenthol. Die Destillationsausbeute am Seitenabzug lag somit oberhalb 99 %.

### Beispiel 2:

Der Trennwandkolonne aus Beispiel 1 wurde in der Kolonnenmitte auf einer Höhe von 331 cm zum Zulaufteil der Trennwand 900 g/h eines auf 105°C vorgewärmten flüssigen L-Menthols synthetischer Herkunft, das durch katalytische Hydrierung von L-Isolulegol an einem Nickel-haltigen Katalysator erhalten wurde, zugeführt, das 99,39 GC-Flächen-% L-Menthol, 0,29 GC-Flächen-% Isopulegol, 0,25 GC-Flächen-% Neomenthol und 0,011 GC-Flächen-% Isomenthol sowie 0,044 GC-Flächen-% Neoisomenthol enthielt. Die Kolonne wurde bei 50 mbar Kopfdruck und einem Rücklauf von 3,0 kg/h betrieben. Dabei stellte sich ein Druckverlust von etwa 35 mbar (± 1 mbar) ein. Am Kopf der Kolonne wurde eine Temperatur von 120°C und im Sumpf eine Temperatur von 135 °C (± 0,5 K) gemessen. Die Kolonne wurde ohne Sumpfabzug betrieben und die Destillatabnahme über eine Waagensteuerung auf 15 g/h (± 1 g/h) eingestellt. Das Rücklaufverhältnis betrug somit etwa 200: 1. Der Kondensator der Kolonne wurde auf 40°C temperiert, um Feststoffbildung zu vermeiden.

Die Flüssigkeit wurde oberhalb der Trennwand in einem Verhältnis von 1 : 1 (Zulauf- : Entnahmeteil) aufgeteilt. In einer Höhe von 300 cm im Abnahmeteil der Trennwand wurde ein gasförmiger Seitenabzug (f) entnommen und in einem Glaskühler kondensiert, aus dem in Abhängigkeit vom Sumpffüllstand etwa 885 bis 890 g/h Reinprodukt über eine Pumpe abgezogen wurde.

Das am Seitenabzug gewonnene Reinprodukt enthielt neben 99,93 GC-Flächen % L-Menthol auch 0,027 GC-Flächen-% Neomenthol und Spuren anderer Menthol-Diastereomere. Das auch bei Raumtemperatur flüssige Destillat enthielt 73,1 GC-Flächen-% L-Menthol, 13,5 GC-Flächen-% Isopulegol, 10,9 GC-Flächen-% Neo-Menthol und 1,79 GC-Flächen-% Neoisomenthol. Der kontinuierlich betriebenen Kolonne wurde innerhalb 24,5 h mit 22,05 kg Zulauf zugeführt und 21,6 kg Reinprodukt am Seitenabzug entnommen. Die Destillationsausbeute am Seitenabzug lag somit oberhalb 98,5 %.

Vergleichsbeispiel:
In einer mit 1 m Sulzer DX-Packung ausgerüsteten Glas-Laborkolonne (theoretische Stufenzahl von etwa 20) mit einem Innendurchmesser von 50 mm, die mit einer Blase und einem umgepumpten Dünnschichtverdampfer (0,05m²) ausgerüstet ist, wurden 614 g eines synthetisch hergestellten L-Menthols mit 98,0 GC.-Flächen % L-Menthol, 1,69 GC.-Flächen % Isopulegol und 0,33 GC.-Flächen % Neomenthol batchweise bei einem Kopfdruck von 50 mbar destilliert. Der Kondensator der Kolonne wurde mit auf 40°C temperiertem Wasser betrieben.

Die Temperaturen am Kopf der Kolonne lagen zwischen 122 und 123 °C, die Sumpftemperatur zwischen 124°C zu Beginn und 125°C gegen Ende der Destillation. Der Destillatbehälter wurde dabei auf etwa 60°C elektrisch beheizt, um ein Festwerden der Fraktion zu verhindern. Bei einem Rücklaufverhältnis von 15:1 wurden 3 Fraktionen (31, 45 und 138 g) sowie bei einem Rücklaufverhältnis von 10:1 eine weitere DestillatFraktion von 116 g gewonnen. Die erste gewonnene Fraktion enthielt 75,5 GC-Flächen-% L-Menthol, 19,6 GC-Flächen-% Isopulegol und 3,01 GC-Flächen-% Neo-menthol und blieb auch bei Raumtemperatur flüssig. Die zweite Fraktion enthielt 90,6 GC-Flächen-% Menthol, 7,03 GC-Flächen-% Isopulegol und 1,49 GC-Flächen-% Neomenthol, die dritte entsprechend 98,09 GC-Flächen-% L-Menthol, 0,98 GC-Flächen-% Isopulegol und 0,3 GC-Flächen-% Neomenthol. In der vierten Fraktion wurde schließlich eine Menthol-Reinheit von 99,52 GC-Flächen-% erreicht. Aus der Blase wurden 197 g Rückstand isoliert, mit 98,5 GC-Flächen-% L-Menthol.

## Patentansprüche

1. Kontinuierliches Verfahren zur Herstellung von racemischem oder optisch aktivem Menthol der Formel (I) 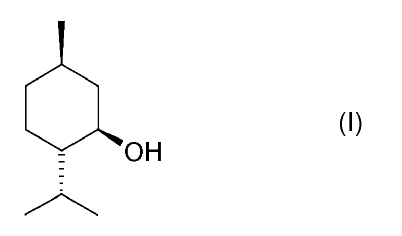 in reiner oder angereicherter Form durch destillative Abtrennung von racemischem oder optisch aktivem Menthol aus Stoffgemischen enthaltend racemisches oder optisch aktives Menthol und Diastereomere des Menthols wobei man die destillative Abtrennung in einer Trennwandkolonne mit 50 bis 300 theoretischen Trennstufen und einer oder mehrerer Seitenabzugsstellen bei einem absoluten Betriebsdruck von 5 bis 500 mbar durchführt.

2. Verfahren nach Anspruch 1 zur Herstellung von racemischem oder optisch aktivem Menthol in reiner oder angereicherter Form durch destillative Abtrennung von racemischem oder optisch aktivem Menthol aus Stoffgemischen enthaltend racemisches oder optisch aktives Menthol und Diastereomere des Menthols sowie Isopulegol der Formel (II) 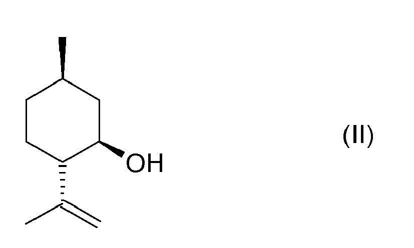 und/oder dessen Diastereomere und gegebenenfalls Menthone der Formel (III) und/oder (IV)

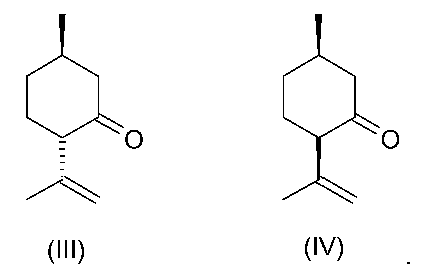

3. Verfahren nach Anspruch 1 oder 2 zur Herstellung von L-Menthol in reiner oder angereicherter Form durch destillative Abtrennung von L-Menthol aus Stoffgemischen enthaltend L-Menthol und Diastereomere des Menthols.

4. Verfahren nach einem der Ansprüche 1 bis 3, **dadurch gekennzeichnet, dass** man ein Stoffgemisch mit einem auf das gesamte Gemisch bezogenen Menthol-Gehalt von 85 bis 99,9 Gew.-% einsetzt.

5. Verfahren nach einem der Ansprüche 1 bis 4, **dadurch gekennzeichnet, dass** man Menthol mit einem Gehalt von 99,5 bis 99,95 Gew.-% erhält.

6. Verfahren nach einem der Ansprüche 1 bis 5, **dadurch gekennzeichnet, dass** das erhaltene Menthol in reiner oder angereicherter Form einen Gehalt an Isopulegol und dessen Diasteromeren von zusammen bis zu 0,5 Gew.-% aufweist.

7. Verfahren nach einem der Ansprüche 1 bis 6, **dadurch gekennzeichnet, dass** das erhaltene Menthol in reiner oder angereicherter Form einen Gehalt an Menthon und Isomenthon von zusammen bis zu 0,5 Gew.-% aufweist.

8. Verfahren nach einem der Ansprüche 1 bis 7, **dadurch gekennzeichnet, dass** man Menthol enthaltende Stoffgemische einsetzt, die durch Hydrierung von Isopulegol erhalten wurden.

9. Verfahren nach einem der Ansprüche 1 bis 8, **dadurch gekennzeichnet, dass** man die destillative Abtrennung in der Trennwandkolonne bei einem absoluten Betriebsdruck von 20 bis 100 mbar durchführt.

10. Verfahren nach einem der Ansprüche 1 bis 9, **dadurch gekennzeichnet, dass** man die destillative Trennung in einer Trennwandkolonne mit 100 bis 180 theoretischen Stufen durchführt.

11. Verfahren nach einem der Ansprüche 1 bis 10, **dadurch gekennzeichnet, dass** man die Trennwandkolonne bei einem absoluten Kopfdruck von 20 bis 60 mbar betreibt.

12. Verfahren nach einem der Ansprüche 1 bis 11, **dadurch gekennzeichnet, dass** man die Trennwandkolonne bei einem absoluten Sumpfdruck von 50 bis 100 mbar betreibt.

13. Verfahren nach einem der Ansprüche 1 bis 12, **dadurch gekennzeichnet, dass** man eine Trennwandkolonne mit einer Seitenabzugsstelle einsetzt.

14. Verfahren nach einem der Ansprüche 1 bis 13, **dadurch gekennzeichnet, dass** man als Trennwandkolonne eine Packungskolonne mit geordnete Blech- oder Gewebepackungen mit einer spezifischen Oberfläche von etwa 100 bis 750 m²/m³ einsetzt.

15. Verfahren nach einem der Ansprüche 1 bis 14, **dadurch gekennzeichnet, dass** man Menthon und/oder Isomenthon enthaltende Stoffgemische einsetzt.

16. Verfahren nach einem der Ansprüche 1 bis 15, **dadurch gekennzeichnet, dass** man eine Trennwandkolonne (TK) einsetzt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4) sowie eines Entnahmeteils (3, 5) mit Abtriebsteil (3) und Verstärkungsteil (5) aufweist.

17. Verfahren nach einem der Ansprüche 1 bis 16, **dadurch gekennzeichnet, dass** man das als Einsatzstoff dienende Menthol enthaltende Stoffgemisch (a) in den mittleren Bereich des Zulaufteils (2, 4) zuführt, das Menthol in reiner oder angereicherter Form als Seitenabzug aus dem mittleren Bereich des Entnahmeteils (3, 5) gewinnt und eine oder mehrere Leichtsiederfraktionen aus dem oberen gemeinsamen Kolonnenbereich (1) und eine oder mehrere Hochsiederfraktionen aus dem unteren gemeinsamen Kolonnenbereich (6) abführt.

18. Verfahren nach einem der Ansprüche 1 bis 17 **dadurch gekennzeichnet, dass** die Trennwandkolonne mit einem Fallfilmverdampfer ausgerüstet wird und eine Hochsiederfraktion in flüssiger Form hinter dem Fallfilmverdampfer ausgeschleust wird.

19. Verfahren nach einem der Ansprüche 1 bis 18, **dadurch gekennzeichnet, dass** der Kondensator und/oder der Nachkondensator der Trennwandkolonne als ein in den Kolonnenkopf integrierter Plattenapparat ausgeführt ist.

20. Verfahren nach einem der Ansprüche 1 bis 19, **dadurch gekennzeichnet, dass** das zur Kühlung des Kondensators und/oder des Nachkondensators der Trennwandkolonne eingesetzte Wärmeträgermedium in einem Temperaturbereich von 0 bis 60 °C temperierbar ist.

21. Verfahren nach einem der Ansprüche 1 bis 20 **dadurch gekennzeichnet, dass** alle mit der Trennwandkolonne verbundenen produktführenden Leitungen, Behälter und Apparate sowie alle Apparate und Leitungen des Vakuumsystems thermisch isoliert und mit einem temperierbaren Begleitheizungssystem ausgerüstet sind.
